Europäisches Patentamt

(19)     European Patent Office

Office européen des brevets

(11)     **EP 0 680 350 B1**

(12)     **FASCICULE DE BREVET EUROPEEN**

(45) Date de publication et mention
de la délivrance du brevet:
**13.05.1998  Bulletin 1998/20**

(21) Numéro de dépôt: **93913103.3**

(22) Date de dépôt: **09.06.1993**

(51) Int. Cl.$^6$: **A61M 16/00**

(86) Numéro de dépôt international:
**PCT/FR93/00547**

(87) Numéro de publication internationale:
**WO 93/25260 (23.12.1993  Gazette 1993/30)**

(54) **APPAREIL D'AIDE A LA RESPIRATION NOTAMMENT POUR TRAITER L'APNEE DU SOMMEIL**

HILFSGERäT FüR DIE BEATMUNG, INSBESONDERE FüR DIE BEHANDLUNG DES
SCHLAFATEMSTILLSTANDES

BREATHING AID APPARATUS PARTICULARLY FOR TREATING SLEEP APNOEA

(84) Etats contractants désignés:
**AT BE CH DE DK ES FR GB IE IT LI NL SE**

(30) Priorité: **15.06.1992 FR 9207184**

(43) Date de publication de la demande:
**08.11.1995  Bulletin 1995/45**

(73) Titulaire:
**NELLCOR PURITAN BENNETT FRANCE
DEVELOPPEMENT
91975 Courtabouef-Les Ulis Cédex (FR)**

(72) Inventeur: **BOURDON, Guy
F-91370 Verrières-le-Buisson (FR)**

(74) Mandataire: **Pontet, Bernard
Pontet & Allano s.a.r.l.
25, rue Jean-Rostand
Parc Club Orsay Université
91893 Orsay Cédex (FR)**

(56) Documents cités:
**EP-A- 0 505 232            WO-A-88/10108
WO-A-89/10768            WO-A-90/14121
US-A- 5 117 819**

## Description

La présente invention concerne un appareil d'aide à la respiration, notamment pour traiter les personnes sujettes à l'apnée du sommeil.

Le syndrome d'apnée du sommeil (SAS), est l'ensemble des signes ainsi que leurs conséquences dus à l'interruption périodique de la respiration pendant le sommeil. Le rétablissement de la respiration ne s'effectue généralement que lorsque la personne concernée se réveille. Ce phénomène peut se produire plusieurs centaines de fois par nuit, avec des interruptions de 10 secondes ou plus à chaque fois.

Il y a trois types de syndrome d'apnée du sommeil, chacun correspondant à une pathologie particulière.

Le premier type, le plus fréquent, est l'apnée obstructive. Il résulte d'une obstruction des voies aériennes supérieures provoquée par un affaissement de la langue et du palais. Les mouvements respiratoires continuent, mais à cause de cette obstruction, l'air ne peut ni entrer ni sortir des poumons.

Le second type, plus rare, est appelé "apnée centrale". Elle se produit lorsque le centre respiratoire du cerveau ne commande plus la respiration. En l'absence de signal en provenance du cerveau, les muscles respiratoires ne fonctionnent pas et l'air ne peut ni entrer ni sortir des poumons.

Le troisième type est l'apnée mixte qui est une combinaison des deux types précédents, le début de l'apnée étant de type central.

Dans le cas de l'apnée obstructive et de l'apnée mixte, le traitement par pression positive continue est le plus couramment utilisé. Cette technique consiste à appliquer en permanence, par l'intermédiaire d'un masque nasal relié par un tuyau à un appareil générateur de pression, une faible pression relative positive dans les voies aériennes supérieures afin d'éviter leur obstruction. Cette pression empêche la langue et le palais de se coller l'un à l'autre. Le résultat est immédiat: une respiration ininterrompue est rétablie, les poumons reçoivent l'oxygène dont ils ont besoin et la personne dort beaucoup mieux.

La valeur optimale de la pression correspond au minimum permettant de supprimer les apnées et les désaturations en oxygène qui en résultent dans le sang.

La détermination de cette pression optimale est réalisée au laboratoire, en soumettant le malade à un enregistrement polygraphique, et en montant progressivement le niveau de pression appliqué au patient jusqu'à disparition des événements respiratoires.

Le traitement décrit précédemment, consistant à appliquer au patient pendant toute la nuit un niveau de pression constant, présente un certain nombre de lacunes.

En effet, la fréquence et l'importance des apnées varient, au cours de la nuit, selon le stade de sommeil dans lequel se trouve le patient. Elles évoluent également dans le temps en fonction de l'évolution de l'état du patient (prise ou perte de poids, absorption d'alcool avant le coucher...).

Ainsi, la pression de traitement déterminée à la prescription n'est pas forcément adéquate par la suite. Or, des enregistrements de contrôle ne peuvent pas être réalisés régulièrement, à cause de leur coût et de la charge importante des laboratoires du sommeil, liée au grand nombre de patients à traiter.

De plus, le patient subit pendant toute la nuit une pression identique, alors que suivant les stades de son sommeil, une pression inférieure peut être suffisante, ou une pression supérieure nécessaire. Or, plus la pression moyenne appliquée pendant la nuit sera basse, meilleurs seront le confort du patient et donc son acceptation du traitement, et plus les effets délétères liés à une pression trop forte seront minimisés.

L'invention vise ainsi un appareil d'aide à la respiration, notamment pour traiter l'apnée du sommeil, comprenant des moyens pour produire un flux de gaz respirable sous une légère pression relative positive, des moyens pour guider ce flux jusqu'à un masque respiratoire, des moyens d'acquisition d'un paramètre représentatif de l'activité respiratoire du patient, et des moyens de réglage automatique pour augmenter la pression appliquée au moins lorsque le paramètre représentatif est indicatif d'une hypopnée, et réduire la pression appliquée lorsque le paramètre représentatif est indicatif d'une respiration normale pendant une durée prédéterminée.

Le terme "hypopnée" englobe les phénomènes de disparition totale de la respiration, et peut aussi englober certains phénomènes de disparition partielle de la respiration, dus à une obstruction partielle des voies aériennes supérieures.

Un tel appareil est connu d'après le WO-A-9014121, selon lequel on applique au patient une pression respiratoire que l'on fait varier au cours du cycle respiratoire pour lui donner une valeur maximale au début de l'inspiration dans le but d'effectuer une sorte d'ouverture forcée des voies respiratoires à ce stade du cycle. En outre, il est prévu de modifier la pression appliquée en fonction de l'activité respiratoire. Ce dispositif est complexe et coûteux.

Dans un appareil connu d'après le WO-A-8810108, on règle la vitesse d'un compresseur de soufflage en fonction de l'activité respiratoire.

Dans ces deux appareils antérieurs, pour effectuer le réglage, on détecte l'activité respiratoire d'après ses effets, notamment sonores, sur l'environnement. Cet art antérieur se base sur le fait que les apnées ou les hypopnées sont fréquemment annoncées par une période de ronflement respiratoire.

Une telle détection est complexe à mettre en oeuvre, peu précise et sujette à des dysfonctionnements. En particulier, elle est peut-être opérante pour détecter le ronflement, mais si l'apnée ou l'hypopnée n'est pas précédée par un tel symptôme annonciateur, l'ensemble du dispositif est inefficace pour corriger la pression appliquée.

Le but de la présente invention est ainsi de proposer un appareil d'aide à la respiration qui soit à la fois simple, plus économique et plus fiable.

Suivant l'invention, l'appareil d'aide à la respiration est caractérisé en ce que les moyens d'acquisition du paramètre représentatif sont des moyens d'acquisition de l'amplitude de variations induites par l'activité respiratoire du patient sur une variable de fonctionnement de l'appareil.

Selon l'invention, on exploite le fait que l'activité respiratoire du patient, lorsqu'elle existe, induit des variations sur au moins un paramètre de fonctionnement de l'appareil. Par exemple, la pression dans le masque, si elle n'est pas régulée, est plus forte lorsque le patient expire que lorsqu'il inspire.

Plus l'activité respiratoire du patient est intense et plus l'amplitude de ces variations cycliques est importante.

C'est pourquoi il est prévu selon l'invention de détecter l'amplitude desdites variations pour augmenter la pression lorsque cette amplitude devient inférieure à un certain seuil en-dessous duquel on considère qu'il y a anomalie respiratoire.

L'invention utilise donc un critère clair et facile à exploiter pour détecter les anomalies.

De préférence, l'appareil selon l'invention comprend en outre des moyens de régulation du fonctionnement d'une source de gaz respirable pour tendre à ramener automatiquement la pression d'écoulement du flux de gaz à une valeur de consigne qui est la même pour les phases d'inspiration et d'expiration, et les moyens de réglage automatique sont des moyens pour modifier la valeur de consigne de la pression, tandis que la variable dont l'amplitude de variation est captée par les moyens d'acquisition du paramètre représentatif est une variable qui varie lorsque les moyens de régulation modifient le fonctionnement de la source pour maintenir la pression constante.

Selon cette version préférée de l'invention, on régule à une valeur sensiblement constante la pression appliquée pendant tout le cycle respiratoire. On élimine ainsi la surpression qui tend à s'établir lorsque le malade expire "contre" la pression produite par l'appareil. Il résulte de cette régulation une variation cyclique de l'activité de l'appareil respiratoire, avec une activité plus forte pendant l'inspiration et réduite pendant l'expiration. Cette variation cyclique d'activité peut être détectée sur la base de différentes variables de fonctionnement de l'appareil, par exemple vitesse de la turbine si la source de gaz respiratoire est un compresseur, débit de gaz respiratoire envoyé au patient, et....

En outre, le réglage effectué, consistant en une variation de la consigne de pression, est facile à mettre en ouvre avec précision.

De préférence, la pression ne peut pas descendre endessous d'un seuil bas défini par le prescripteur et réglé sur l'appareil, et bien sûr elle ne peut pas dépasser la valeur maximale que l'appareil est capable de délivrer, ou une valeur maximale définie par le médecin.

D'autres particularités et avantages de l'invention ressortiront encore de la description ci-après, relative à des exemples non-limitatifs.

Aux dessins annexés :

- la figure 1 est un schéma d'un appareil selon l'invention ;
- la figure 2 est un organigramme pour le fonctionnement du calculateur de la figure 1 ;
- les figures 3 et 4 sont des schémas analogues à la figure 1 mais relatifs à deux autres modes de réalisation ; et
- la figure 5 est un organigramme de fonctionnement du calculateur du mode de réalisation de la figure 4.

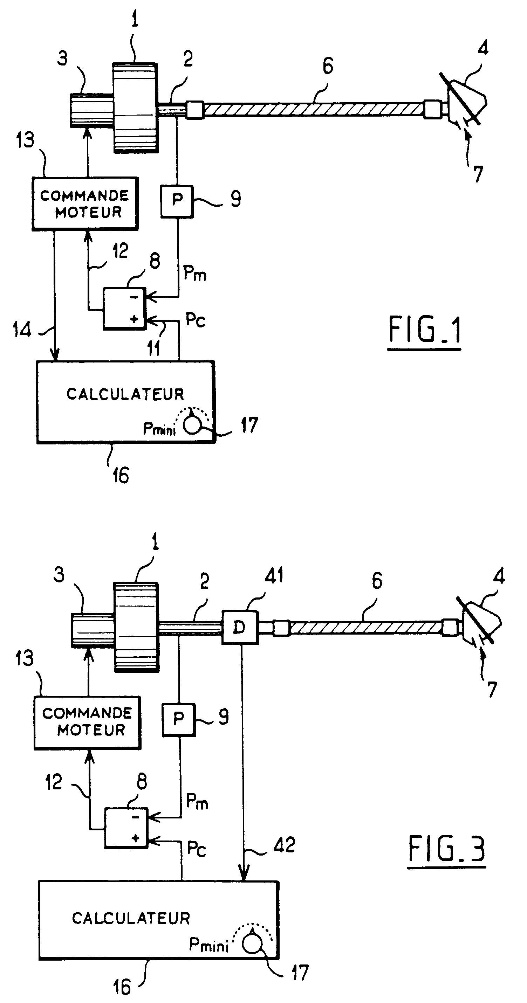

L'appareil représenté à la figure 1 comprend un compresseur 1 capable de produire à travers son orifice de refoulement 2 un gaz respirable sous une pression positive relative, c'est à dire mesurée par rapport à la pression atmosphérique. Cette pression positive dépend de la vitesse de rotation du moteur d'entraînement 3 du compresseur 1. De manière non représentée, le compresseur 1 est du type produisant la pression relative positive par une turbine de propulsion du gaz respirable. L'orifice de refoulement 2 est relié à un masque nasal 4 par un tuyau souple 6. Le masque nasal 4 est destiné à être appliqué sur le visage du patient, par exemple au moyen d'une sangle. Le masque 4 comporte un orifice 7 permettant au patient d'expirer malgré l'écoulement en sens contraire provenant du compresseur 1.

Un comparateur 8 compare en permanence la pression $P_m$ détectée dans l'orifice de refoulement 2 du compresseur 1 par un détecteur de pression 9, avec une consigne de pression $P_c$ appliquée à l'autre entrée 11 du comparateur 8. En fonction du résultat de la comparaison, le comparateur 8 fournit sur sa sortie 12 un signal appliqué à un dispositif 13 de commande du moteur pour réduire la vitesse de rotation du moteur 3 lorsque la pression mesurée par le détecteur 9 est supérieure à la consigne de pression, et pour augmenter la vitesse de rotation du moteur 3 et donc la pression à l'orifice de refoulement 2 lorsque la pression mesurée par le détecteur 9 est inférieure à la consigne de pression.

Ainsi, la pression à l'orifice de refoulement 2, et donc dans le masque nasal 4, est sensiblement la même pendant les phases d'inspiration et pendant les phases d'expiration du patient.

Pendant les phases d'inspiration, une dépression relative tend à se créer à l'orifice de refoulement 2 du compresseur 1, et le maintien de la pression à la valeur de consigne exige d'augmenter la vitesse de rotation du moteur 3.

Au contraire, pendant les phases d'expiration du patient, il tend à se créer une surpression excessive à l'orifice de refoulement 2, et le maintien de la pression à la valeur de consigne exige de réduire la vitesse de rotation du moteur 3.

Par conséquent, lorsque la respiration du patient est normale, la vitesse de rotation du moteur 3 suit une courbe périodique.

Suivant le mode de réalisation selon la figure 1, un signal représentatif de la vitesse de rotation du moteur 3 est appliqué par le dispositif de commande 13 à une entrée 14 d'un calculateur 16 qui a pour fonction d'analyser la courbe de vitesse du moteur 3 en tant que paramètre représentatif de l'activité respiratoire du patient, et de modifier la consigne de pression $P_c$ appliquée à l'entrée 11 du comparateur 8 en fonction du résultat de cette analyse.

D'une manière générale, lorsque l'analyse de la courbe de vitesse de rotation du moteur révèle une situation d'hypopnée, le calculateur 16 augmente la consigne de pression.

Au contraire, si l'analyse de la courbe de vitesse du moteur révèle une absence d'hypopnée pendant une certaine durée prédéterminée, le calculateur réduit d'un pas prédéterminé la consigne de pression.

Le calculateur 16 est relié à une commande manuelle 17 permettant de régler la pression de consigne minimale $P_{mini}$ autorisée par le médecin à chaque patient.

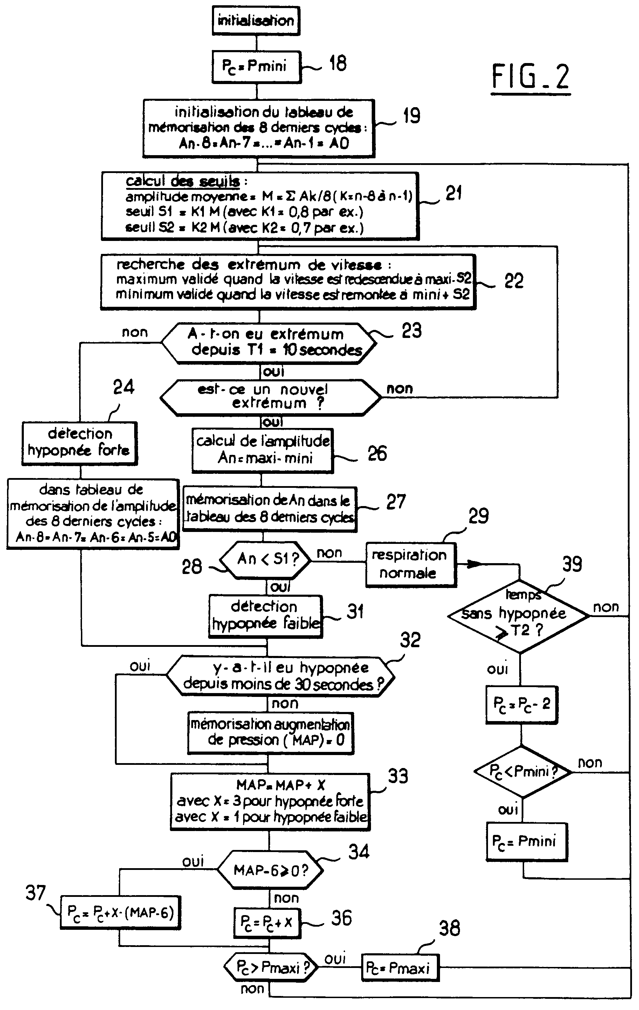

On va maintenant décrire en référence à la figure 2, l'organigramme selon lequel, pour l'essentiel, est programmé le calculateur 16.

Dans la suite, on appelle "hypopnée" le symptôme consistant soit en une diminution anormale (par exemple de 50%) de l'activité respiratoire, soit le symptôme d'apnée totale consistant en la disparition totale de l'activité respiratoire.

Au départ, la pression de consigne Pc est choisie égale à $P_{mini}$, c'est-à-dire la pression de consigne minimale choisie à l'aide de la commande manuelle 17 (étape 18).

A l'étape 19, on rend arbitrairement égales à une valeur AO, relativement faible, les valeurs An-8, An-7, ..., An-1 de l'amplitude de la variation de vitesse du moteur lors des huit cycles respiratoires précédant celui qui est en cours d'analyse.

Ensuite, à l'étape 21, on calcule la moyenne des amplitudes des huit cycles précédents (moyenne M), et on calcule deux seuils S1 et S2 avec par exemple:

$$S1 = 0,8\,M$$

$$S2 = 0,7\,M$$

A l'étape 22, on recherche les extrémums de vitesse de rotation du moteur.

Pour cela, la vitesse de rotation du moteur à chaque cycle d'exécution du programme est mise en mémoire. Un maximum ou un minimum n'est validé que si la vitesse a ensuite varié suffisamment pour revenir en-deçà de ce maximum ou minimum d'une valeur au moins égale au seuil S2.

En d'autres termes, comme le seuil S2 est plus grand que la moitié de la moyenne des amplitudes antérieures, un extremum donné ne sera pris en compte que si la vitesse repasse ensuite de l'autre côté de la valeur moyenne des vitesses. En particulier, si la respiration s'arrête (apnée totale), la vitesse du moteur prend sa valeur moyenne et l'extrémum précédent n'est pas validé. Plus généralement, s'il tend à s'établir une amplitude inférieure au seuil S2, les extrémums ne pourront plus être validés.

Au bout d'une durée T1 égale par exemple à 10 secondes, ceci est détecté au test suivant 23. En l'absence d'extremum pendant 10 secondes, on passe par une branche "détection hypopnée forte" 24 de l'organigramme, dans laquelle on ramène à la valeur relativement basse AO les quatre amplitudes An-8 ... An-5 les plus anciennes encore en mémoire. Ceci a pour but de réduire les seuils S1 et S2 au prochain cycle de calcul, de manière à déceler plus facilement une reprise de l'activité respiratoire.

En revenant au test 23, si un extremum a été trouvé dans les 10 secondes qui précèdent et si cet extremum est le même que celui déjà pris en compte lors du précédent cycle de calcul, on retourne à l'étape 22 de recherche des extremums.

Si au contraire l'extremum est nouveau, on passe par une étape 26 de calcul de la nouvelle amplitude An, puis, étape 27, mise en mémoire de l'amplitude An avec effacement simultané de la plus ancienne des amplitudes en mémoire An-8.

A l'étape 28, on compare l'amplitude nouvelle-ment calculée An avec le plus grand S1 des deux seuils.

Si l'amplitude nouvellement calculée An est supérieure au seuil S1, on passe sur une branche 29 respiration normale qui sera décrite plus loin.

Dans le cas contraire, c'est-à-dire si l'amplitude est comprise entre les seuils S1 et S2, on considère qu'il y a hypopnée faible 31.

Que l'on ait enregistré une hypopnée forte 24 ou une hypopnée faible 31, on effectue un test 32 pour savoir s'il y a déjà eu une hypopnée dans les 30 secondes qui ont précédé. Dans la négative, on remet à zéro le nombre MAP qui correspond à l'augmentation totale de pression dans les 30 secondes qui ont précédé.

Si au contraire il y a eu hypopnée dans les 30 secondes qui précèdent, on ne remet pas à zéro le nombre MAP.

L'étape suivante 33 consiste à ajouter un incrément relativement fort au nombre MAP si on a détecté une hypopnée forte, et un incrément relativement faible si on a détecté une hypopnée faible. Puis, étape 34, on teste si le nouveau nombre MAP dépasse 6 cm d'eau (6hPa). Dans la négative, étape 36, on ajoute l'incrément x, fort ou faible selon la force de l'hypopnée, à la consigne de pression Pc. Si au contraire MAP excède 6, on n'augmente la pression de consigne $P_c$ que dans une mesure telle que l'augmentation totale dans les 30 secondes qui précèdent soit égale à 6 (étape 37).

Ceci a pour but d'éviter d'augmenter outre mesure la pression pour traiter une seule hypopnée: si une augmentation de plus de 6 cm d'eau est nécessaire pour traiter une hypopnée, c'est qu'il y a une anomalie quelconque et il vaut mieux que le patient se réveille.

Ensuite, la nouvelle pression de consigne est appliquée au comparateur 8 de la figure 1 sous réserve qu'elle n'excède pas la pression de consigne maximum $P_{maxi}$. Si la pression $P_c$ excède $P_{maxi}$, la consigne appliquée au comparateur 8 est égale à $P_{maxi}$ (étape 38). On est ensuite ramené à l'étape 21 du calcul des seuils. Si l'hypopnée forte ou faible qui a été détectée lors du cycle précédent n'est toujours pas levée, la pression de consigne va être augmentée d'un nouvel incrément et ainsi de suite jusqu'à ce que l'augmentation totale de la pression MAP dans les 30 secondes atteigne 6 cm d'eau ou jusqu'à ce que l'hypopnée soit levée.

Ainsi, on compare l'amplitude à deux seuils différents, l'un pour détecter les hypopnées fortes, incluant les apnées totales, et appliquer une augmentation relativement rapide de la pression de consigne, l'autre pour détecter les hypopnées faibles, résultant d'une obstruction partielle des voix respiratoires supérieures, et appliquer une augmentation de pression nettement plus modérée.

L'une des particularités importantes de l'invention consiste à analyser le paramètre représentatif de l'activité respiratoire (la vitesse du moteur 3) non pas par comparaison avec des seuils absolus, mais par comparaison avec l'activité respiratoire qui a juste précédé l'anomalie respiratoire. On a en effet constaté que l'activité respiratoire variait beaucoup au cours du sommeil, si bien qu'une activité qui serait normale dans une certaine phase de sommeil peut correspondre à une hypopnée dans une autre phase du sommeil.

Revenant à la branche 29 de l'organigramme, celle-ci conduit à un test 39 pour déterminer si un temps T2 s'est écoulé sans détection d'hypopnée. Dans la négative, on retourne à l'étape 21 de calcul des seuils.

Si au contraire un temps T2, par exemple égal à 30 minutes, s'est écoulé sans hypopnée, on réduit la pression de consigne de par exemple 2 cm d'eau. On se donne ainsi une chance de ramener la pression appliquée au malade à une valeur inférieure si cela est possible.

Toutefois, si la nouvelle pression de consigne devenait ainsi inférieure à la pression minimale telle qu'affichée grâce à la commande manuelle 17 de la figure 1, la pression de consigne est simplement rendue égale à la pression mini affichée. On est ensuite, une fois encore, ramené à l'étape 21 de calcul des seuils.

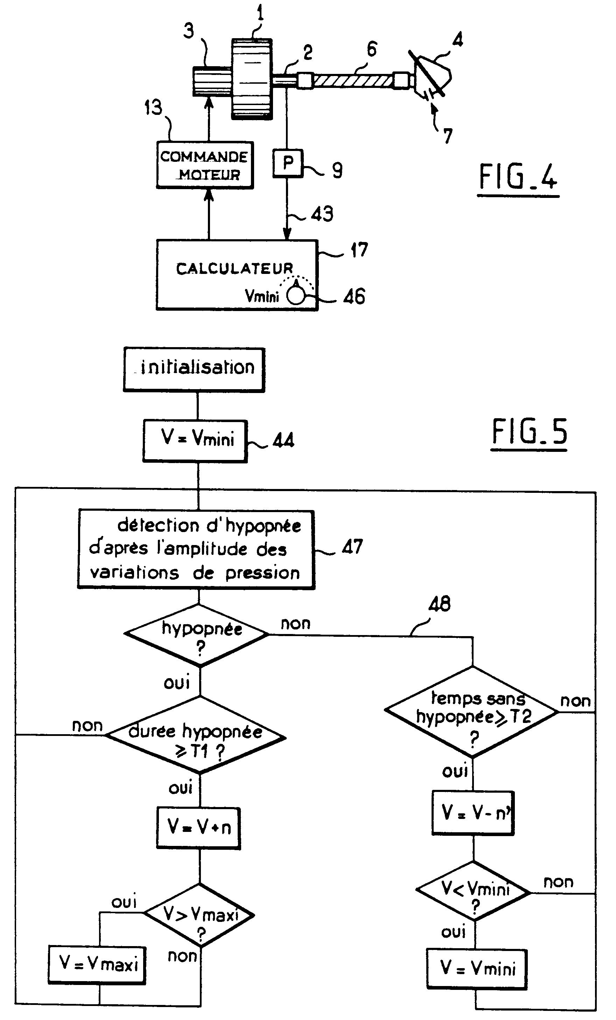

Dans l'exemple représenté à la figure 3, qui ne sera décrit qu'en ce qui concerne ses différences par rapport à celui de la figure 1, on a placé sur l'orifice de refoulement 2 du compresseur 1 un détecteur de débit 41 dont le signal est envoyé à une entrée 42 du calculateur. Par contre, le calculateur ne reçoit plus de signal correspondant à la vitesse de rotation du moteur. C'est maintenant le signal de débit fourni par le détecteur 41 qui donne au calculateur le paramètre représentatif de l'activité respiratoire. Lorsque le malade inspire, le détecteur de débit 41 relève un débit plus élevé que lorsque le malade expire. Autrement dit, les variations du débit se font en sens contraire de celles de la vitesse du moteur 3. A part cela, rien n'est changé, et l'organigramme de la figure 2 est valable pour le mode de réalisation de la figure 3, excepté que, à l'étape 22 de recherche des extremums, il faut remplacer le mot "vitesse" par le mot "débit".

L'exemple de la figure 4 correspond à une version simplifiée.

Dans cet exemple, qui ne sera décrit qu'en ce qui concerne ses différences par rapport à celui de la figure 1, il n'y a pas de régulation de pression à l'orifice de refoulement 2, c'est-à-dire que, en dehors des situations d'apnée ou d'hypopnée, le moteur 3 tourne à la même vitesse lorsque le patient inspire et lorsqu'il expire. La pression à l'orifice de refoulement 2 est donc relativement basse lorsque le malade inspire et relativement forte lorsqu'il expire. La pression à l'orifice de refoulement 2 constitue donc un paramètre représentatif de l'activité respiratoire et elle est, à ce titre, détectée par le capteur de pression 9. Le calculateur 16, qui reçoit sur une entrée 43 le signal de pression 9, analyse la courbe de pression et fournit au dispositif de commande 13 du moteur 3 un signal pour augmenter la vitesse du moteur 3 lorsque les variations de la pression sont indicatives d'une situation d'hypopnée, et pour réduire la vitesse du moteur 3 lorsqu'aucune situation d'hypopnée n'a été relevée pendant une durée déterminée, par exemple 30 minutes.

La figure 5 représente un organigramme schématique selon lequel peut être programmé le calculateur 17 de la figure 4.

Au départ, la vitesse V du moteur est réglée à une valeur $V_{mini}$ (étape 44) affichée grâce à une commande manuelle 46 (figure 4).

On passe ensuite à une étape 47 de détection des hypopnées d'après l'amplitude des variations de pression. Cette étape peut correspondre aux étapes 21 et 22 de la figure 2, sauf qu'elle est alors appliquée à la pression au lieu d'être appliquée à la vitesse du moteur. En l'absence d'hypopnée, on passe par une branche 48 dans laquelle on réduit d'une valeur prédéterminée n' la vitesse du moteur si un temps T2, par exemple de 30 minutes, s'est écoulé sans hypopnée, sans toutefois faire tomber la vitesse à une valeur inférieure à la vitesse affichée $V_{mini}$.

En cas d'hypopnée détectée pendant une durée supérieure ou égale à une valeur $T_1$ de par exemple 10 secondes, on incrémente la vitesse V d'une valeur prédéterminée n, sans toutefois laisser la vitesse dépasser une valeur $V_{maxi}$.

Par conséquent, dans cet exemple simplifié, on ne distingue qu'un seul degré d'intensité de l'hypopnée et lorsque l'hypopnée est détectée, il est prévu un seul et même mode d'action pour tous les cas, c'est-à-dire une incrémentation de la vitesse du moteur selon un pas prédéterminé et un seul.

Bien entendu, l'invention n'est pas limitée aux exemples décrits et représentés.

On pourrait prévoir dans les calculateurs des réalisations selon les figures 1 et 3 un programme ne distinguant qu'un seul type d'hypopnée, ou au contraire équiper. Le mode de réalisation selon la figure 4 avec un programme traitant de manière différente les hypopnées faibles et les hypopnées fortes comme cela a été décrit en référence à la figure 2.

**Revendications**

1. Appareil d'aide à la respiration, notamment pour traiter l'apnée du sommeil, comprenant des moyens (1, 3) pour produire un flux de gaz respirable sous une légère pression relative positive, des moyens (6) pour guider ce flux jusqu'à un masque respiratoire (4), des moyens (14, 26, 42, 43) d'acquisition d'un paramètre (An) représentatif de l'activité respiratoire du patient, et des moyens de réglage automatique pour augmenter la pression appliquée au moins lorsque le paramètre représentatif est indicatif d'une hypopnée, et réduire la pression appliquée lorsque le paramètre représentatif est indicatif d'une respiration normale pendant une durée prédéterminée ($T_2$), caractérisé en ce que les moyens d'acquisition du paramètre représentatif sont des moyens d'acquisition de l'amplitude (An) de variations induites par l'activité respiratoire du patient sur une variable de fonctionnement de l'appareil.

2. Appareil selon la revendication 1, caractérisé en ce que les moyens de réglage automatique sont des moyens pour régler la vitesse de fonctionnement d'un moteur (3) d'un compresseur (1) de production du flux de gaz respirable.

3. Appareil selon la revendication 1 ou 2, caractérisé en ce que les moyens d'acquisition du paramètre représentatif sont des moyens d'acquisition de l'amplitude des variations de la pression (P) du flux de gaz (figure 4).

4. Appareil selon la revendication 1, caractérisé en ce qu'il comprend en outre des moyens (8, 13) de régulation du fonctionnement d'une source (1) de gaz respirable pour tendre à ramener automatiquement la pression d'écoulement du flux de gaz à une valeur de consigne ($P_c$) qui est la même pour les phases d'inspiration et d'expiration, en ce que les moyens de réglage automatique sont des moyens (16) pour modifier la valeur de consigne de la pression, et en ce que la variable dont l'amplitude de variation est captée par les moyens d'acquisition du paramètre représentatif est une variable qui varie lorsque les moyens de régulation modifient le fonctionnement de la source pour maintenir la pression constante.

5. Appareil selon la revendication 1, 2 ou 4, caractérisé en ce que les moyens d'acquisition du paramètre représentatif sont des moyens d'acquisition de l'amplitude (An) de la variation d'un paramètre indicatif de l'écoulement du gaz respirable.

6. Appareil selon la revendication 5, caractérisé en ce que les moyens d'acquisition d'un paramètre représentatif comprennent des moyens (41, 42) de détection du débit d'écoulement du gaz respirable.

7. Appareil selon la revendication 5, caractérisé en ce que les moyens d'acquisition d'un paramètre représentatif comprennent des moyens (14) de détection d'un paramètre de fonctionnement d'un compresseur de production du flux de gaz respirable.

8. Appareil selon l'une des revendications 1 à 7, caractérisé en ce qu'il comprend des moyens de comparaison (22, 28) pour comparer l'amplitude de ladite variation avec au moins un seuil (S1, S2) calculé d'après une valeur d'amplitude (M) portant sur au moins une période de variation antérieure, et en ce que les moyens de réglage automatique augmentent la pression lorsque l'amplitude (An) est inférieure au seuil.

9. Appareil selon la revendication 8, caractérisé en ce que le seuil (S1, S2) est calculé d'après une valeur d'amplitude moyenne (M) portant sur plusieurs périodes antérieures (An-8, ... An-1).

10. Appareil selon l'une des revendications 1 à 7, caractérisé en ce qu'il comprend des moyens pour comparer l'amplitude de ladite variation avec un seuil de prise en compte (S2) et en ce que les moyens de réglage automatique augmentent la pression appliquée lorsque l'amplitude (An) reste inférieure au seuil de prise en compte pendant une seconde durée prédéterminée (T1).

11. Appareil selon l'une des revendications 1 à 7, caractérisé en ce qu'il comprend des moyens pour comparer le paramètre représentatif (An) avec un seuil (S1) de faible hypopnée et un seuil (S2) de forte hypopnée, en ce que les moyens de réglage automatique augmentent la pression appliquée au moyen d'un premier réglage incrémentiel (x), relativement grand, lorsque le paramètre est au-delà du seuil (S2) de forte hypopnée, par rapport au seuil (S1) de faible hypopnée, et d'un deuxième réglage incrémentiel (x), relativement petit, lorsque le paramètre représentatif est compris entre les deux seuils (S1, S2).

12. Appareil selon l'une des revendications 8 à 11, caractérisé par des moyens (24) pour réduire le seuil après détection d'une hypopnée.

13. Appareil selon l'une des revendications 1 à 12, caractérisé en ce que les moyens de réglage sont conçus pour augmenter la pression (Pc) par réglages incrémentiels successifs (x) lorsque le paramètre représentatif (An) conserve une valeur indicative d'une hypopnée.

14. Appareil selon la revendication 13, caractérisé en ce qu'il comprend des moyens (34, 37) pour limiter l'augmentation de pression (MAP) permise pendant un intervalle de temps prédéterminé.

**Claims**

1. Breathing aids apparatus, in particular for treating sleep apnoea, comprising means (1, 3) for producing a flow of breathable gas under a low positive relative pressure, means (6) for leading this flow to a respiratory mask (4), means (14, 26, 42, 43) of acquiring a parameter (An) representative of the respiratory activity of the patient, and automatic adjustment means for increasing the pressure applied at least when the representative parameter is indicative of a hypopnoa, and for reducing the applied pressure when the representative parameter is indicative of normal respiration over a predetermined time (T2), characterized in that the means of acquiring a representative parameter are means of acquiring the amplitude (An) of variations induced by the respiratory activity of the patient upon an operation variable of the apparatus.

2. An apparatus according to claim 1, characterized in that said automatic adjustment means are means for adjusting the speed of a motor (3) of a compressor (1) for production of the flow of breathable gas.

3. An apparatus according to claim 1 or 2, characterized in that the means of acquiring a representative parameter are means for acquiring the amplitude of the variations of the pressure (P) in said flow of breathable gas (figure 4).

4. An apparatus according to claim 1, charaterized in that it furthermore comprises means (8, 13) for controlling operation of a source (1) of breathable gas in order to tend to automatically bring the flow pressure in said flow of breathable gas to a set point value ($P_c$) which is the same for inspiration and expiration phases, in that said automatic adjustment means are means (16) for modifying said set point value of the pressure, and in that said variable, the amplitude of which is acquired by the acquisition means of the representative parameter is a variable which varies when the control means apply modifications to the operation of the source in order to maintain the pressure at a constant magnitude.

5. An apparatus according to claim 1, 2 or 4 characterized in that said means of acquiring the representative parameter are means for acquiring the amplitude (An) of the variation of a parameter which is indicative of the flow of breathable gas.

6. An apparatus according to claim 5, characterized in that said means of acquiring a representative parameter comprise means (41, 42) for detecting the flow rate of the breathable gas.

7. An apparatus according to claim 5, characterized in that said means of acquiring a representative parameter comprise means (14) for detecting an operation parameter of a compressor producing the flow of breathable gas.

8. An apparatus according to one of claim 1 to 7, characterized in that it comprises comparison means (22, 28) for comparing the amplitude of said variation with at least one threshold (S1, S2) calculated from at least one amplitude value (M) relating to at least one previous variation period, and in that said automatic adjustment means automatically increase the applied pressure when the amplitude (An) is below the threshold.

9. An apparatus according to claim 8, characterized in that said threshold (S1, S2) is calculated from an average amplitude value (M) relating to several prior periods (An-8,...An-1).

10. An apparatus according to one of claims 1 to 7, characterized in that it comprises means for comparing the amplitude of said variation with a validation threshold (S2) and in that the automatic adjustment means increase the applied pressure when the amplitude (An) remains below the validation threshold for a second predetermined duration time (T1).

11. An apparatus according to one of claim 1 to 7, characterized in that it comprises means for comparing the representative parameter (An) with a threshold (S1) for weak hypopnoea and a threshold (S2) for strong hypopnoea, in that said automatic adjustment means increase the applied pressure by a first incremental adjustment (x), which is relatively large, when the parameter is beyond the threshold (S2) for strong hypopnoa, relative to the threshold (S1) for weak hypopnoea, and by a second incremential adjustment (x), which is relatively small, when the parameter is comprised between the two thresholds (S1, S2).

12. An apparatus according to one of claims 8 to 11, characterized by means (24) for reducing the threshold after detection of a hypopnoea.

13. An apparatus according to one of claims 1 to 12, characterized in that the adjustment means are adapted to increase the pressure (Pc) by successive incremential adjustments (x) when the representative parameter (An) retains a value indicative of a hypopnoea.

14. An apparatus according to claim 13, characterized by comprising means (34, 37) for limiting the increase in pressure (MAP) permitted for a predetermined time interval.

**Patentansprüche**

1. Atemhilfegerät, insbesondere für die Behandlung des Atemstillstands wahrend des Schlafens, mit Mitteln (1, 3) für die Erzeugung eines Flusses eines atembaren Gases unter einem leichten relativen positiven Druck, Mitteln (6), um diesen Fluß bis zu einer Atemmaske (4) zu leiten, Mitteln (14, 26, 42, 43) für die Erfassung eines Parameter (An), der für die Atemtatigkeit des Patienten repräsentativ ist, und Mitteln für die automatische Einstellung zur Erhöhung des angewandten Drucks, zumindest wenn der repräsentative Parameter eine zu geringe Atemtätigkeit anzeigt, und zur Verringerung des angewandten Drucks, wenn der repräsentative Parameter eine normale Atmung während einer vorbestimmten Dauer ($T_2$) anzeigt, dadurch gekennzeichnet, daß die Mittel für die Erfassung des repräsentativen Parameters Mittel zur Erfassung der Amplitude (An) von Änderungen sind, die durch die Atemtätigkeit des Patienten bei einer Funktionsvariablen des Geräts erzeugt werden.

2. Gerät nach Anspruch 1, dadurch gekennzeichnet, daß die Mittel zur automatischen Einstellung Mittel zur Einstellung der Betriebsgeschwindigkeit eines Motors (3) eines Kompressors (1) zur Erzeugung des Flusses des atembaren Gases sind.

3. Gerät nach Anspruch 1 oder 2, dadurch gekennzeichnet, daß die Mittel zur Erfassung des repräsentativen Parameters Mittel zur Erfassung der Amplitude der Änderungen des Drucks (P) des Gasflusses sind (Figur 4).

4. Gerät nach Anspruch 1, dadurch gekennzeichnet, daß es ferner Mittel (8, 13) zur Regelung des Betriebs einer Quelle (1) von atembarem Gas umfaßt, um zu versuchen, automatisch den Fließdruck des Gasflusses auf einen Sollwert ($P_c$) zu bringen, der für die Einatmungsphasen und die Ausatmungsphasen derselbe ist, daß die Mittel zur automatischen Einstellung Mittel (16) sind, um den Drucksollwert zu verändern, und daß die Variable, deren Veränderungsamplitude von den Mitteln zur Erfassung des repräsentativen Parameters erfaßt wird, eine Variable ist, die sich ändert, wenn die Mittel zur Regelung den Betrieb der Quelle verändern, um den Druck konstant zu halten.

5. Gerät nach Anspruch 1, 2 oder 4, dadurch gekennzeichnet, daß die Mittel zur Erfassung des repräsentativen Parameters Mittel zur Erfassung der Amplitude (An) der Veränderung eines Parameters sind, der das Fließen des atembaren Gases anzeigt.

6. Gerät nach Anspruch 5, dadurch gekennzeichnet, daß die Mittel zur Erfassung eines repräsentativen Parameters Mittel (41, 42) für die Erfassung der Fließmenge des atembaren Gases umfassen.

7. Gerät nach Anspruch 5, dadurch gekennzeichnet, daß die Mittel zur Erfassung eines repräsentativen Parameters Mittel (14) zur Erfassung eines Betriebsparameters eines Kompressors zur Erzeugung des Flusses des atembaren Gases umfassen.

8. Gerät nach einem der Ansprüche 1 bis 7, dadurch gekennzeichnet, daß es Vergleichsmittel (22, 28) umfaßt, um die Amplitude der Veränderung mit mindestens einem Grenzwert (S1, S2) zu vergleichen, der nach einem Amplitudenwert (M), der sich auf mindestens einen vorhergehenden Veränderungs periode bezieht, berechnet wird, und daß die Mittel zur automatischen Einstellung den Druck erhöhen, wenn die Amplitude (An) kleiner als der Grenzwert ist.

9. Gerät nach Anspruch 8, dadurch gekennzeichnet, daß der Grenzwert (S1, S2) nach einem durchschnittlichen Amplitudenwert (M) berechnet wird, der sich auf mehrere vorhergehende Perioden (An-8, .... An-1) bezieht.

10. Gerät nach einem der Ansprüche 1 bis 7, dadurch gekennzeichnet, daß es Mittel für den Vergleich der Amplitude der Veränderung mit einem Berücksichtigungsgrenzwert (S2) umfaßt und daß die Mittel zur automatischen Einstellung den angewandten Druck erhöhen, wenn die Amplitude (An) unter dem Berücksichtigungsgrenzwert während eines zweiten vorbestimmten Zeitraumes (T1) bleibt.

11. Gerät nach einem der Ansprüche 1 bis 7, dadurch gekennzeichnet, daß es Mittel für den Vergleich des repräsentativen Parameters (An) mit einem Grenzwert (S1) für geringfügig zu schwache Atmung und einen Grenzwert (S2) für stark zu schwache Atmung umfaßt, daß die Mittel zur automatischen Einstellung den angewandten Druck mit Hilfe einer ersten Inkrementaleinstellung (x), die relativ groß ist, erhöhen, wenn der Parameter jenseits der Schwelle (S2) der stark abgeschwächten Atmung im Vergleich zu der Schwelle (S1) der geringfügig zu schwachen Atmung liegt, und einer zweiten Inkrementaleinstellung (x), die relativ klein ist, wenn der repräsentative Parameter zwischen den beiden Grenzwerten (S1, S2) liegt.

12. Gerät nach einem der Ansprüche 8 bis 11, gekennzeichnet durch Mittel (24) zur Verringerung des Grenzwertes nach Erfassung einer zu schwachen Atmung.

13. Gerät nach einem der Ansprüche 1 bis 12, dadurch gekennzeichnet, daß die Mittel zur Einstellung derart ausgeführt sind, daß sie den Druck (Pc) durch aufeinanderfolgende Inkrementaleinstellungen (x) erhöhen, wenn der repräsentative Parameter (An) einen auf eine zu schwache Atmung hinweisenden Wert beibehält.

14. Gerät nach Anspruch 13, dadurch gekennzeichnet, daß es Mittel (34, 37) umfaßt, um die Druckerhöhung (MAP), die während eines vorbestimmten Zeitraumes gestattet ist, zu begrenzen.

EP 0 680 350 B1

FIG.1

FIG.3

10

## FIG.2

```
initialisation
```
↓
$P_C = Pmini$ — 18
↓
initialisation du tableau de
mémorisation des 8 derniers cycles :
An-8 = An-7 = ... = An-1 = A0 — 19
↓
**calcul des seuils :**
amplitude moyenne = $M = \Sigma\, Ak/8\,(K=n-8\,à\,n-1)$
seuil S1 = K1 M (avec K1 = 0,8 par ex.)
seuil S2 = K2 M (avec K2 = 0,7 par ex.) — 21
↓
recherche des extrémum de vitesse :
maximum validé quand la vitesse est redescendue à maxi - S2
minimum validé quand la vitesse est remontée à mini + S2 — 22
↓
A-t-on eu extrémum
depuis T1 = 10 secondes — 23    → non
↓ oui
est-ce un nouvel
extrémum ?    → non
↓ oui

24 → détection
hypopnée forte

calcul de l'amplitude
An = maxi - mini — 26
↓
dans tableau de
mémorisation de l'amplitude
des 8 derniers cycles :
An-8 = An-7 = An-6 = An-5 = A0

mémorisation de An dans le
tableau des 8 derniers cycles — 27
↓
28 — An < S1 ?    → non → respiration normale — 29
↓ oui
détection
hypopnée faible — 31
↓
y-a-t-il eu hypopnée
depuis moins de 30 secondes ? — 32    → oui
↓ non
mémorisation augmentation
de pression (MAP) = 0
↓
MAP = MAP + X — 33
avec X = 3 pour hypopnée forte
avec X = 1 pour hypopnée faible
↓
MAP - 6 ≥ 0 ? — 34    → oui
↓ non
37 → $P_C = P_C + X \cdot (MAP-6)$     $P_C = P_C + X$ — 36
↓
$P_C > Pmaxi$ ?    → oui → $P_C = Pmaxi$ — 38
↓ non

39
temps
sans hypopnée
> T2 ?    → non
↓ oui
$P_C = P_C - 2$
↓
$P_C < Pmini$ ?    → non
↓ oui
$P_C = Pmini$
```

$\underline{\text{FIG.4}}$

$\underline{\text{FIG.5}}$

détection d'hypopnée
d'après l'amplitude des
variations de pression ~ 47

hypopnée ?

non ~ 48

oui

durée hypopnée ⩾ T1 ?

non

oui

V = V+n

V > Vmaxi ?

oui

non

V = Vmaxi

temps sans hypopnée ⩾ T2 ?

non

oui

V = V-n'

V < Vmini ?

non

oui

V = Vmini

initialisation

V = Vmini ~ 44